# EUROPÄISCHE PATENTSCHRIFT

(11) **EP 4 684 754 B1**
(45) Veröffentlichungstag und Bekanntmachung des Hinweises auf die Patenterteilung: **01.07.2026**
(21) Anmeldenummer: 24191154.4
(22) Anmeldetag: 26.07.2024
(51) Int. Cl.: A61B 90/50, A61B 50/28, F16M 11/04, F16M 11/08, F16M 13/02

(54) **FALLSICHERUNG FÜR MEDIZINISCHEN TRAGARM**
FALL PROTECTION FOR MEDICAL SUPPORT ARMS
PROTECTION CONTRE LA CHUTE POUR BRAS DE SUPPORT MÉDICAL

(43) Veröffentlichungstag der Anmeldung: 28.01.2026
(73) Patentinhaber: MAVIG GmbH, 81829 München (DE)
(72) Erfinder: MURATIDI, Georg, 36280 Oberaula (DE); HOFFSTETTER, Marc, 80634 München (DE); ENGLERT, Raphael, 36251 Bad Hersfeld (DE); ZAPF, Lukas, 81927 München (DE); BÜTTNER, Alexander, 81829 München (DE)
(74) Vertreter: Vossius & Partner Patentanwälte Rechtsanwälte mbB

(56) Entgegenhaltungen:
- WO-A1-01/11916
- DE-A1- 10 051 898
- GB-A- 2 475 298
- US-A1- 2022 128 193

## Beschreibung

Die vorliegende Offenbarung betrifft eine Fangvorrichtung zur Sicherung einer Aufhängeinrichtung für medizinische Geräte und insbesondere eine Fallsicherung für medizinische Tragarme.

In ärztlichen Praxen und Kliniken sowie medizinisch-technische Arbeitsplätze in medizinischen Labors werden Gebrauchsgegenstände in aufgehängten Gebrauchsstellungen im Bereich von Arbeitsplätzen mittels Tragarmen gehalten. Solche als Aufhängeeinrichtungen dienende Tragarme werden insbesondere an medizinischen Arbeitsplätzen verwendet, medizinische Gebrauchsgegenstände, wie beispielsweise Röntgenabschirmungen, Messgeräte, Bildwiedergabegeräte (Monitore), Ablageteile für Arbeits- oder Behandlungswerkzeuge und dergleichen bereitzuhalten.

Aufgrund des teilweise hohen Gewichts der mittels des Tragarms bereitgestellten Gebrauchsgegenstände und der häufigen Positions- und Höhenverstellung der zumeist mehrteiligen und langen Ausleger kann es zu Ermüdungserscheinungen und nachfolgend Brüchen an Schwachstellen kommen. Dazu zählen insbesondere Gelenkverbindungen und Aufhängungspunkte. Um Unfälle und Verletzungen des sich im Bereich der Aufhängeeinrichtung aufhaltenden Personenkreises zu verhindern, muss daher sichergestellt sein, dass die Tragarme bei Brüchen so gesichert ist, dass die abbrechenden Teile nicht bis zum Boden stürzen können.

Die DE 100 51 898 A1 beschreibt eine Fangvorrichtung für eine Fangvorrichtung zur Sicherung von an einer Aufhängeeinrichtung aufgehängten medizinischen Gebrauchsgegenständen gegen Herabstürzen. Die Fangvorrichtung umfasst eine Sicherungsschlinge, die zur Verbindung mit der Aufhängeeinrichtung um einen Bolzen der Aufhängeeinrichtung schlingbar ist, wobei die Sicherungsschlinge Verbreiterungen an ihren Enden aufweist, die in einem Rückhaltekörper ausgebildete Öffnungen durchgreifen. Weiterhin umfasst die Fangvorrichtung einen Hauptkörper, der mit einem Sicherungsbolzen eines Verbindungselements, welches an dem Gebrauchsgegenstand fixiert ist, durch einen Sicherungsstift verbindbar ist. Der Hauptkörper ist mit dem Rückhaltekörper lösbar verbindbar.

Due US 2022/0128193 A1 beschreibt eine Auffangvorrichtung für ein Trägersystem zur vorzugsweise beweglichen Halterung von mindestens einem medizinischen Gerät, mit mindestens einer Trägervorrichtung, die mindestens einen kritischen Punkt aufweist, und mindestens einem länglichen Sicherungsteil. Die Trägervorrichtung weist eine erste Durchgangsöffnung in einem ersten Bereich, der von dem kritischen Punkt beabstandet ist, und eine zweite Durchgangsöffnung in einem zweiten Bereich auf, der dem ersten Bereich in Bezug auf den kritischen Punkt gegenüberliegt und von dem kritischen Punkt beabstandet ist. Das Sicherungsteil ist durch beide Durchgangsöffnungen geführt, und die Auffangvorrichtung umfasst ein erstes Blockierelement, das an einem ersten Ende des Sicherungsteils befestigt ist, und ein zweites Blockierelement, das an einem zweiten Ende des Sicherungsteils , das dem ersten Ende des Sicherungsteils gegenüberliegt, befestigt ist. Die beiden Sperrelemente sind derart angeordnet, dass ein Herausgleiten des Sicherungsteils aus den beiden Durchgangsöffnungen blockiert ist.

Es ist eine Aufgabe der vorliegenden Offenbarung, eine verbesserte Fallsicherung für einen Tragarm zur Aufnahme eines medizinischen Geräts bereitzustellen, die sicher den Absturz des angebauten Geräts verhindern kann. Ferner soll die Offenbarungsgemäße Fallsicherung konstruktiv einfach und insbesondere im Vergleich zu den bekannten Lösungen kostengünstig herzustellen sein.

Die vorliegende Offenbarung ist im Patentanspruch 1 definiert; die abhängigen Ansprüche beschreiben Ausführungsformen der Offenbarung.

Durch die Offenbarung wird ein Tragarm zur Aufnahme eines medizinischen Geräts bereitgestellt. Der Tragarm weist an einem seiner Enden ein Gelenk mit einer Bohrung auf, die eingerichtet ist, einen Zapfen aufzunehmen, so dass der Zapfen in der Bohrung rotierbar ist. Der Zapfen ist dazu eingerichtet, das medizinische Gerät zu Halten und ist insbesondere mit dem medizinischen Gerät verbunden. Das Gelenk weist eine Einkerbung als Sollbruchstelle auf. Der Tragarm weist ferner mit einem Seil auf, das an einem seiner Enden einen Haken aufweist, der in eine Öffnung des Gelenks einhakbar ist. Das Seil ist vorzugsweise ein Stahlseil. Die Einkerbung ist zwischen der Öffnung und der Bohrung angeordnet. Das andere Ende des Seils ist mit dem Zapfen verbindbar, so dass im verbundenen Zustand das Seil innerhalb der Bohrung verläuft. Das Seil weist eine Einrichtung auf, die eingerichtet ist, ein Verdrillen des Seils bei einer Drehung des Zapfens in der Bohrung zu verhindern.

Durch den Aufbau des Offenbarungsgemäßen Tragarms kann das Seil eine mechanische Verbindung zwischen dem Tragarm und dem medizinischen Gerät sicherstellen.

Der Zapfen ist vorzugsweise axial in die Bohrung einschiebbar. Vorzugsweise ist der Haken ein Karabinerhaken.

Der Zapfen ist insbesondere zum Halten einer Röntgenabschirmung, eines Messgeräts, eines Bildwiedergabegeräts, eines Monitors), oder eines Ablageteils für Arbeits- oder Behandlungswerkzeuge eingerichtet.

Gemäß einer Ausführungsform kann die Einrichtung zum Verhindern des Verdrillens so ausgebildet sein, dass das Seil mittels einen Stufennippels im Zapfen durch einen Hinterschnitt gehalten wird.

Alternativ kann die Einrichtung zum Verhindern des Verdrillens eine Drehhülse aufweisen. In diesem Fall kann das Seil durch eine Schlaufe mittels eines Bolzens am Zapfen befestigt sein.

Gegenüber der oben genannten DE 100 51 898 A1 ist die Konstruktion des Offenbarungsgemäßen Tragarms einfacher, insbesondere da ein komplexes Frästeil, das in der DE 100 51 898 A1 als Sicherungsbolzen benötigt wird, unnötig ist. Ferner wird gemäß der DE 100 51 898 A1 der Hauptkörper mit dem Sicherungsbolzen eines Verbindungselements, das an dem Gebrauchsgegenstand fixiert ist, durch einen der Stift verbunden. Bei der Montage kann dieser Stift allerdings nicht beobachtet werden. Im Gegensatz dazu kann der der Haken der vorliegenden Offenbarung beim Einhaken in die Öffnung des Gelenks beobachtet werden, was die Sicherheit der Fallsicherung erhöht. Im Falle der Offenbarungsgemäßen Ausführungsform mit Drehhülse bleibt Raum im Zapfen, der beispielsweise zur Durchführung von Kabeln verwendet werden kann.

Im Fall der US 2022/0128193 A1 ist das Seil an den entgegengesetzten Enden des Gelenks befestigt, wobei sie Sollbruchstelle dazwischen liegt. Versagt bei US 2022/0128193 die axiale Sicherung des Zapfens in dem Gelenk oder wird diese axiale Sicherung durch den Monteur versehentlich gelöst bzw. fehlerhaft montiert, kommt es zum Absturz des Anbaugerätes. Im Gegensatz dazu ist bei der vorliegenden Offenbarung ein Ende des Seils am Gelenk angeordnet, wobei das andere Ende am Zapfen des Anbaugerätes befestigt ist. Dazwischen liegt gemäß der Offenbarung nicht nur die Sollbruchstelle, sondern auch die Fixierung des Zapfens im Gelenk. So wird bei einer Trennung des Zapfens und des Gelenks bei der Offenbarung ein Absturz abgefangen, da sich das medizinische Gerät mit dem Zapfen über das Seil und den Haken am Gelenk abstützt.

Die Offenbarung wird im Folgenden unter Bezug auf die Figure näher erläutert, wobei
Fig. 1 schematisch den Aufbau eines Tragarms gemäß der vorliegenden Offenbarung,
Fig. 2 die Verbindung zwischen Tragarm und Zapfen und
Fig. 3 die Anordnung der Einrichtung zum Verhindern des Verdrillens gemäß den Ausführungsformen A und B zeigt.

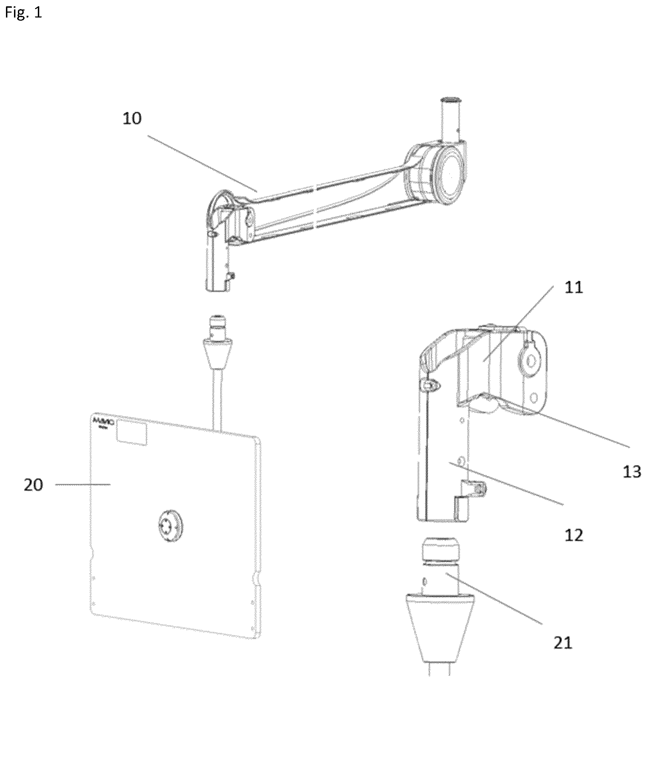

Fig. 1 zeigt schematisch den Aufbau des Offenbarungsgemäßen Tragarms gemäß einer Ausführungsform. Der Tragarm 10 weist in bekannter Art ein Gelenk 11 auf, das an einer Seite mit einem Ausleger verbindbar ist, um das Gelenk beispielsweise schwenkbar an der Wand oder Decke eines Behandlungsraums zu befestigen, und das an der anderen Seite mit einem Zapfen 21 verbindbar ist, der eingerichtet ist, ein medizinisches Gerät 20 zu halten. Zum Verbinden des Zapfens 21 mit dem Gelenkt 11 kann das Gelenk 11 eine Bohrung 12 aufweisen, in die der Zapfen 21 axial eingeschoben und beispielweise durch einen Klickmechanismus axial gehalten werden kann, um rotierbar mit dem Gelenk 11 verbunden zu sein.

Das Gelenk 11 an einer definierten Stelle eine Einkerbung 13 auf, die im Fall einer Überlastung durch das Gewicht des medizinischen Geräts 20 oder durch zu starke mechanische Belastung durch z.B. Kollision eine Sollbruchstelle darstellt. In diesem Fall ist der Tragarm 10 am medizinischen Gerät 20 mittels eines Seils 22, insbesondere eines Stahlseils gegen Herabfallen gesichert, was im Folgenden genauer beschrieben wird.

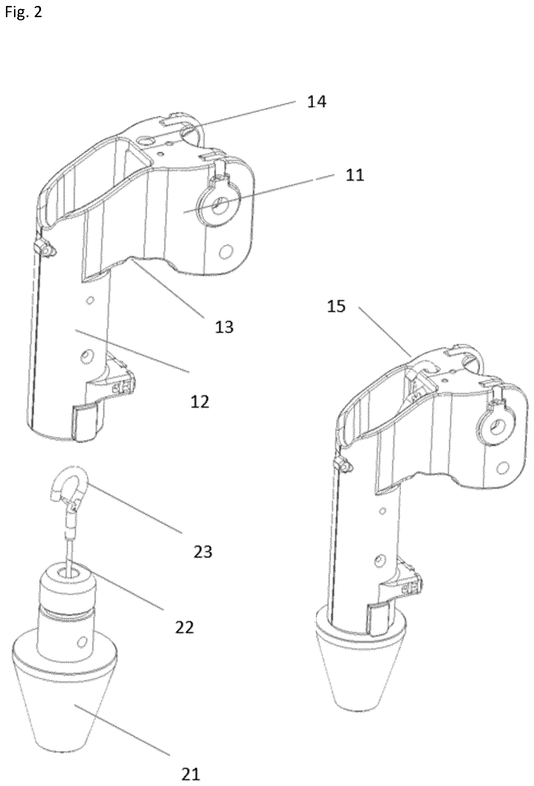

Wie in Fig. 2 gezeigt, ist der Zapfen 21 mit einem Seil 22 verbunden, an dessen Ende sich ein Haken 23, wie der gezeigte Karabinerhaken befindet. Wird der Zapfen 21 in Bohrung 12 gefügt, kann der Haken 23 in eine Öffnung 14 des Gelenks 11 eingehakt werden, wie mit der Bezugsziffer 15 in der Fig. 2 gezeigt. Die Öffnung 14 ist im Gelenk 11 derart angeordnet, dass sich die Einkerbung 13, also die Solbruchstelle zwischen dem Zapfen 21 und der Öffnung 14 zum Einhaken des Hakens 23 befindet, so dass auch bei einem Bruch des Gelenks 11 an der Sollbruchstelle das mit dem Tragarm 10 verbundene Teil des Gelenks 11 mit dem das medizinische Gerät haltenden Zapfen 21 verbunden und gesichert bleibt. Auf diese Weise entsteht also eine mechanische Verbindung zwischen Gerät 20 und Tragarm 10, auch wenn es zu einem Bruch der Sollbruchstelle kommt.

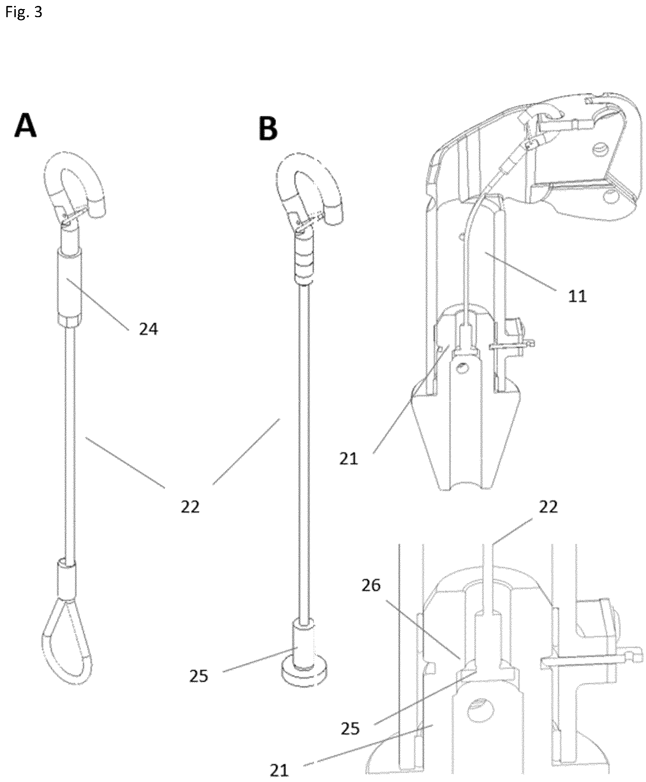

In der Regel ist eine unbegrenzte Drehbarkeit des Gerätes 20 relativ zum Tragarm 10 um den Zapfen 21 gewünscht. Um ein Verdrillen und Abscheren des Seils 22 zu verhindern, muss dieses mit einem rotatorischen Freiheitsgrad ausgestattet sein. Mit Bezug auf Fig. 3 werden zwei Ausführungsformen einer solchen Einrichtung vom Verhindern des Verdrillens beschrieben.

Gemäß der in Fig. 3 gezeigten Ausführungsform A ist am unteren Ende des Seils 22 eine Schlaufe 27 angeordnet, welche beispielsweise mittels eines Bolzens (nicht dargestellt) im Zapfen 21 befestigt ist. Das Seil 22 ist mit einer Drehhülse 24 ausgestattet, welche eine beliebige Verdrehung des Zapfen 21 relativ zur Bohrung 12 des Gelenks 11 erlaubt.

In einer konstruktiv einfacheren und damit günstigeren Ausführungsform B ist das Seil 22 am unteren Ende mit einem Stufennippel 25 versehen. Der Stufennippel 25 wird im Zapfen 21 durch einen Hinterschnitt 26 gehalten. Bei Rotation des Zapfens 21 relativ zum Gelenk 11 kann das Seil 22 mit dem Stufennippel 25 relativ zum Zapfen 21 rotieren. Ein Verdrillen des Stahlseils wird so verhindert.

Bei Überlastung des Gelenks 11 ist durch die Offenbarung sichergestellt, dass das am Zapfen 21 befestigte Gerät 20 gegen Herabfallen gesichert ist, was die Unfallgefahr und die Beschädigung des medizinischen Geräts 20 verhindern bzw. vermindern kann.

Während verschiedene Ausführungsformen der vorliegenden Offenbarung vorstehend beschrieben wurden, sollte es verstanden werden, dass sie nur als Beispiel dargestellt wurden, und nicht als eine Einschränkung. Die verschiedenen Merkmale oder Konfigurationen, die zur Verfügung gestellt werden, sollen dem Fachmann ermöglichen beispielhafte Merkmale und Funktionen der vorliegenden Offenbarung zu verstehen. Der Fachmann verstehen, dass die vorliegende Offenbarung nicht auf die dargestellten Merkmale oder Konfigurationen beschränkt ist, sondern unter Verwendung einer Vielzahl von alternativen Merkmalen und Konfigurationen implementiert werden kann. Darüber hinaus können ein oder mehrere Merkmale einer Ausführungsform mit einem oder mehreren Merkmalen einer anderen der beschriebenen Ausführungsform kombiniert werden, wie ein Fachmann auf dem Gebiet der Technik verstehen würden. Daher sollte die Breite und der Umfang der vorliegenden Offenbarung nicht durch eine der oben beschriebenen beispielhaften Ausführungsformen eingeschränkt werden.

Es versteht sich, dass in der vorliegenden Offenbarung der Begriff "und/oder" oder das Symbol "/" jede und alle Kombinationen von einem oder mehreren der zugehörigen aufgelisteten Elemente umfassen kann. Zum Beispiel umfasst A und/oder B und/oder C jede und alle Kombinationen von einem oder mehreren von A, B und C, einschließlich A, B, C, A und B, A und C, B und C und einer Kombination von A und B und C. Ebenso umfasst A/B/C jede und alle Kombinationen von einem oder mehreren von A, B und C, einschließlich A, B, C, A und B, A und C, B und C und einer Kombination von A und B und C.

Es versteht sich auch, dass jede Bezugnahme auf ein Element hier mit einer Bezeichnung wie "erstes", "zweites" usw. nicht generell die Menge oder Reihenfolge dieser Elemente einschränkt. Vielmehr können diese Bezeichnungen hier als praktisches Mittel zur Unterscheidung zwischen zwei oder mehr Elementen oder Instanzen eines Elements verwendet werden. Der Hinweis auf ein erstes und ein zweites Element bedeutet also nicht, dass nur zwei Elemente verwendet werden können oder dass das erste Element dem zweiten Element in irgendeiner Weise vorausgehen muss.

Verschiedene Modifikationen der in dieser Offenbarung beschriebenen Implementierungen sind für den Fachmann ohne weiteres erkennbar, und die hierin definierten allgemeinen Grundsätze können auf andere Implementierungen angewandt werden, ohne dass dadurch der Anwendungsbereich der Ansprüche verlassen wird. Daher ist die Offenbarung nicht auf die hier gezeigten Implementierungen beschränkt, sondern hat den größtmöglichen Umfang, der mit den hier offengelegten neuen Merkmalen und Prinzipien vereinbar ist, wie sie in den nachstehenden Ansprüchen aufgeführt sind.

## Patentansprüche

1. Tragarm (10) zur Aufnahme eines medizinischen Geräts (20), wobei der Tragarm (10) an einem seiner Enden ein Gelenk (11) mit einer Bohrung (12) aufweist, die eingerichtet ist, einen Zapfen (21) zur Anordnung des medizinischen Geräts (20) aufzunehmen, so dass der Zapfen (21) in der Bohrung (12) rotierbar ist, wobei das Gelenk (11) eine Einkerbung (13) als Sollbruchstelle aufweist,
ferner mit einem Seil (22), das an einem seiner Enden einen Haken (23) aufweist, der in eine Öffnung (14) des Gelenks (11) einhakbar ist, wobei
die Einkerbung (13) zwischen der Öffnung (14) und der Bohrung (12) angeordnet ist, wobei das andere Ende des Seils (22) mit dem Zapfen (21) verbindbar ist, so dass im verbundenen Zustand das Seil (22) innerhalb der Bohrung verläuft,
wobei das Seil (22) eine Einrichtung aufweist, die eingerichtet ist, ein Verdrillen des Seils (22) bei einer Drehung des Zapfens (21) in der Bohrung (12) zu verhindern.

2. Tragarm (10) nach Anspruch 1, wobei das Seil (22) mittels einen Stufennippels (25) im Zapfen (21) durch einen Hinterschnitt (26) gehalten wird.

3. Tragarm (10) nach Anspruch 1, wobei die Einrichtung zum Verhindern des Verdrillens eine Drehhülse (24) aufweist.

4. Tragarm (10) nach Anspruch 3, wobei das Seil (22) eine Schlaufe (27) aufweist, die mittels eines Bolzens am Zapfen (21) befestigt ist.

5. Tragarm (10) nach einem der vorstehenden Ansprüche, wobei der Zapfen (21) axial in die Bohrung (12) einschiebbar ist.

6. Tragarm (10) nach einem der vorstehenden Ansprüche, wobei der Haken (23) ein Karabinerhaken ist.

7. Tragarm (10) nach einem der vorstehenden Ansprüche, wobei durch das Seil (22) eine mechanische Verbindung zwischen dem Tragarm (10) und dem medizinischen Gerät (20) sichergestellt wird.

8. Tragarm (10) nach einem der vorstehenden Ansprüche, wobei das Seil (22) ein Stahlseil ist.

9. Tragarm (10) nach einem der vorstehenden Ansprüche, wobei der Zapfen (21) zum Halten einer Röntgenabschirmung, eines Messgeräts, eines Bildwiedergabegeräts, eines Monitors, oder eines Ablageteils für Arbeits- oder Behandlungswerkzeuge eingerichtet ist.

## Claims

1. A support arm (10) for receiving a medical device (20), wherein the support arm (10) comprises at one of its ends a joint (11) with a bore (12) which is adapted to receive a pivot (21) for the arrangement of the medical device (20), so that the pivot (21) can be rotated in the bore (12), wherein the joint (11) comprises a notch (13) as a predetermined breaking point,
further comprising a rope (22) which has a hook (23) at one of its ends, which hook can be hooked into an opening (14) of the joint (11), wherein
the notch (13) is arranged between the opening (14) and the bore (12), wherein the other end of the rope (22) can be connected to the pivot (21), so that in the connected state the rope (22) extends within the bore,
wherein the rope (22) comprises a means which is adapted to prevent twisting of the rope (22) when the pivot (21) rotates in the bore (12).

2. The support arm (10) according to claim 1, wherein the rope (22) is held by means of a stepped nipple (25) in the pivot (21) by an undercut (26).

3. The support arm (10) according to claim 1, wherein the means for preventing twisting comprises a rotating sleeve (24).

4. The support arm (10) according to claim 3, wherein the rope (22) comprises a loop (27) which is attached to the pivot (21) by means of a bolt.

5. The support arm (10) according to any one of the preceding claims, wherein the pivot (21) can be axially inserted into the bore (12).

6. The support arm (10) according to any one of the preceding claims, wherein the hook (23) is a snap hook.

7. The support arm (10) according to any one of the preceding claims, wherein a mechanical connection between the support arm (10) and the medical device (20) is ensured by the rope (22).

8. The support arm (10) according to any one of the preceding claims, wherein the rope (22) is a steel rope.

9. The support arm (10) according to any one of the preceding claims, wherein the pivot (21) is adapted to hold an X-ray shield, a measuring device, an image reproduction device, a monitor, or a storage part for working or treatment tools.

## Revendications

1. Bras support (10) destiné à recevoir un dispositif médical (20), le bras support (10) présentant, à l'une de ses extrémités, une articulation (11) avec un alésage (12), qui est configuré pour recevoir un tourillon (21) destiné à l'agencement du dispositif médical (20), de sorte que le tourillon (21) soit rotatif dans l'alésage (12), l'articulation (11) présentant une encoche (13) en tant que point de rupture prédéterminé,
comprenant en outre un câble (22), qui présente, à l'une de ses extrémités, un crochet (23), qui peut être accroché dans une ouverture (14) de l'articulation (11),
l'encoche (13) étant disposée entre l'ouverture (14) et l'alésage (12), l'autre extrémité du câble (22) pouvant être reliée au tourillon (21), de sorte qu'à l'état relié le câble (22) s'étende à l'intérieur de l'alésage,
le câble (22) présentant un dispositif qui est configuré pour empêcher une torsion du câble (22) lors d'une rotation du tourillon (21) dans l'alésage (12).

2. Bras support (10) selon la revendication 1, dans lequel le câble (22) est maintenu dans le tourillon (21) au moyen d'un embout étagé (25) par une contre-dépouille (26).

3. Bras support (10) selon la revendication 1, dans lequel le dispositif destiné à empêcher la torsion présente une douille rotative (24).

4. Bras support (10) selon la revendication 3, dans lequel le câble (22) présente une boucle (27), qui est fixée au tourillon (21) au moyen d'un boulon.

5. Bras support (10) selon l'une quelconque des revendications précédentes, dans lequel le tourillon (21) peut être inséré axialement dans l'alésage (12).

6. Bras support (10) selon l'une quelconque des revendications précédentes, dans lequel le crochet (23) est un mousqueton.

7. Bras support (10) selon l'une quelconque des revendications précédentes, dans lequel une liaison mécanique entre le bras support (10) et le dispositif médical (20) est assurée par le câble (22).

8. Bras support (10) selon l'une quelconque des revendications précédentes, dans lequel le câble (22) est un câble en acier.

9. Bras support (10) selon l'une quelconque des revendications précédentes, dans lequel le tourillon (21) est configuré pour maintenir un écran de protection contre les rayons X, un appareil de mesure, un appareil de reproduction d'images, un moniteur ou une pièce de support pour des outils de travail ou de traitement.
